(19) Europäisches Patentamt
European Patent Office
Office européen des brevets

(11) EP 0 487 930 B1

(12) EUROPEAN PATENT SPECIFICATION

(45) Date of publication and mention of the grant of the patent:
**24.03.1999 Bulletin 1999/12**

(51) Int Cl.6: **C07D 221/18**, C07D 491/04, A61K 31/435
// (C07D491/04, 317:00, 221:00)

(21) Application number: **91118676.5**

(22) Date of filing: **01.11.1991**

(54) **Benzo[C]phenanthridinium derivatives**

Benzo[c]phenanthridiniumderivaten

Dérivés de benzo[c]phenanthridinium

(84) Designated Contracting States:
**DE ES FR GB IT NL SE**

(30) Priority: **07.11.1990 JP 299844/90**
**11.06.1991 JP 165174/91**
**11.06.1991 JP 165175/91**

(43) Date of publication of application:
**03.06.1992 Bulletin 1992/23**

(73) Proprietor: **NIPPON KAYAKU KABUSHIKI KAISHA**
**Tokyo 102-0071 (JP)**

(72) Inventors:
- **Suzuki, Masanobu**
  **Omiya-shi (JP)**
- **Nakanishi, Takeshi**
  **Yono-shi (JP)**
- **Kogawa, Osamu**
  **Kashiwa-shi (JP)**
- **Ishikawa, Keizou**
  **Iwatsuki-shi (JP)**
- **Kobayashi, Fumiko**
  **Koto-ku, Tokyo (JP)**
- **Ekimoto, Hisao**
  **Kita-ku, Tokyo (JP)**

(74) Representative: **Türk, Gille, Hrabal**
**Brucknerstrasse 20**
**40593 Düsseldorf (DE)**

(56) References cited:
**EP-A- 0 432 630**

- **CHEMICAL AND PHARMACEUTICAL BULLETIN. vol. 33, no. 4, April 1985, TOKYO JP pages 1763 - 1765; MIYOJI HANAOKA ET AL: 'Synthesis of fagaridine,a phenolic Benzo(c)phenanthridine alkaloid'**
- **JOURNAL OF ORGANIC CHEMISTRY. vol. 53, no. 8, 15 April 1988, EASTON US pages 1708 - 1713; SATINDER V. KESSAR ET AL: 'Benzyne cyclisation route to Benzo(c)phenanthridine alkaloids. Synthesis of Chelerythrine,Decarine, and Nitidine'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 487 930 B1

## Description

BACKGROUND OF THE INVENTION

FIELD OF THE INVENTION

The present invention relates to benzo[c]phenanthridinium derivatives of the general formula A', which have antitumor activities and blood platelet aggregation inhibition activities and therefore may be expected as useful medicines. The invention also relates to a pharmaceutical composition containing said derivatives as effective ingredients.

STATEMENT OF THE PRIOR ART

Nowadays, alkylating agents, nucleic acid metabolism antagonists, antibiotics, plant alkaloids and the like have been used as chemotherapeutic drugs for patients with cancers.
It is also known that a thrombosis is caused by the cohesion and the coagulation of blood platelets, and has a relation with cerebral infarcts, cardiac disorders, cancerous DIC, etc., and also with a metastasis of cancers.
2,3-(methylenedioxy)-5-methyl-7-hydroxy-8-methoxy-benzo[c]phenanthridinium chloride or iodide is a known compound described in Chem. Pharm. Bull., 33, 1763 (1985). It is also known from the disclosures of Japanese Patent Application KOKAI Nos. 2-243628 and 3-184916 that this compound has blood platelet aggregation inhibition activities and antitumor activities. Furthermore, Chem. Pharm. Bull., 33, 1763 (1985) describes a method for preparing the compound, wherein use is made of berberine as the starting material, which is treated in more than ten steps to obtain the aimed compound.
Furthermore, 5-methyl-7-hydroxy-8-methoxy-benzo[c]phenanthridinium chloride or hydroxide having the general formula A, wherein M and N each are a hydrogen atom and $X^-$ is $Cl^-$ or $OH^-$, is a known compound described in J. Org. Chem., 53, 1708-1713 (1988).
EP-A-0 432 630 published on 19.06.91 discloses 7-hydroxy-8-methoxy-5-methyl-2,3-methylenedioxybenzo [c]phenanthridinium acid quaternary salts useful as antifumor agents.

SUMMARY THE INVENTION

The present inventors have made many studies on benzo[c]phenanthridinium derivatives having the general formula A':

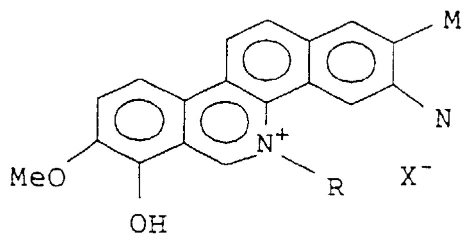

(A')

wherein M and N individually represent a hydroxyl of a $C_1$ - $C_5$ alkoxy group, or M and N simultaneously represent a hydrogen atom or together form a methylenedioxy group; $X^-$ represents $HSO_4^-$ or $H_2PO_4^-$ ; and R represents a $C_1$ - $C_5$ alkyl group.
It has been found that these novel benzo[c]phenanthridinium derivatives have antitumor activities and blood platelet aggregation inhibition activities. The stability of the compounds according to the invention is increased due to their conversion into their $HSO_4^-$ or $H_2PO_4^-$-salts so that such hydrogen salts of the compounds have a merit in preparing pharmaceutical formulation thereof.

DETAILED DESCRIPTION OF THE INVENTION

Therefore, the invention relates to a benzo[c]phenanthridinium derivative having the general formula A':

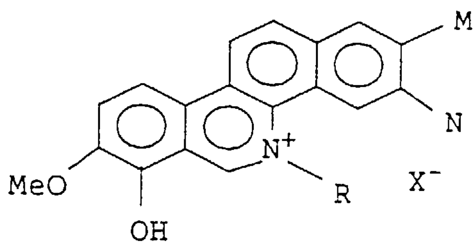

(A')

wherein

M and N individually represent a hydroxyl or $C_1$ - $C_5$ alkoxy group, or M and N simultaneously represent a hydrogen atom or together form a methylenedioxy group;
$X^-$ represents $HSO_4^-$ or $H_2PO_4^-$ ; and R represents a $C_1$ - $C_5$ alkyl group. These compounds can be obtained according to a method, wherein a compound of the general formula C:

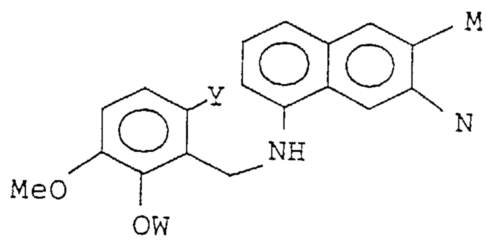

(C)

wherein
M and N individually represent a hydroxyl or $C_1$ - $C_5$ alkoxy group, or M and N simultaneously represent a hydrogen atom or together form a methylenedioxy group;
Y represents a halogen atom; and W represents a protective group, is subjected to a ring closure reaction in the presence of an organic tin compound, and then to an oxidative aromatization reaction to obtain a compound of the general formula D.

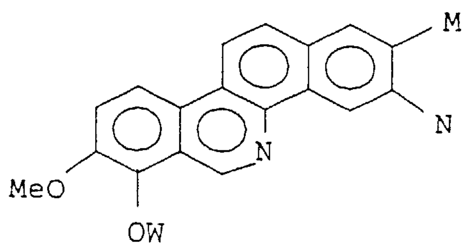

(D)

wherein
M and N individually represent a hydroxyl or $C_1$ - $C_5$ alkoxy group, or M and N simultaneously represent a hydrogen atom or together form a methylenedioxy group;
and W represents a protective group, and
that the compound D thus formed is reacted with an N-alkylating agent to effect an N-alkylation of said compound; and
that the N-alkylated compound thus formed is subjected to a protective group-removing operation and treated with $H_2SO_4$ or $H_3PO_4$ to form the aimed salt type product.

The invention also provides a pharmaceutical composition for the use as an antitumor agent comprising, as effective ingredient, a benzo[c]phenanthridinium derivative of the general formula A':

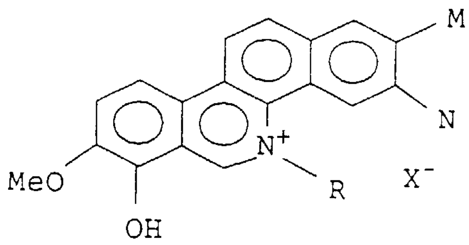

(A')

wherein

M and N individually represent a hydroxyl or $C_1$ - $C_5$ alkoxy group, or M and N simultaneously represent a hydrogen atom or together form a methylenedioxy group;
$X^-$ represents $HSO_4$- or $H_2PO_4^-$ ; and R represents a $C_1$ - $C_5$ alkyl group.

The above-defined hydrogen salts according to the invention have excellent stability, and therefore they have an advantage in formulating a pharmaceutical preparations thereof.

As examples of $C_1$ - $C_5$ alkoxy groups, there may be mentioned methoxy, ethoxy, n-propoxy, i-propoxy, n-butoxy, i-butoxy, t-butoxy and pentoxy groups and the like. Preferred are $C_1$-$C_3$ alkoxy groups such as methoxy, ethoxy, n-propoxy and i-propoxy groups. Examples of $C_1$ - $C_5$ alkyl groups are methyl, ethyl, n-propyl, i-propyl, n-butyl, i-butyl, t-butyl and pentyl groups, etc. Preferred are $C_1$-$C_3$ alkyl groups such as methyl, ethyl, n-propyl and i-propyl groups.

The compounds of the general formula C are novel compounds and may be prepared for instance, in a manner shown below.

The starting material is 2,3-dihydroxy-5-nitronaphthalene which is a known compound [Collection Czechoslovak. Chem. Commun., 53, 3184 (1988)]. The starting material is converted into a nitronaphthalene compound of the formula F:

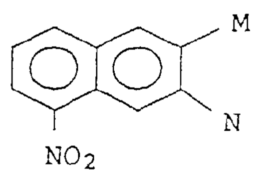

(F)

wherein M and N individually represent a hydroxyl or $C_1$ - $C_5$ alkoxy group, or M and N together form a methylenedioxy group.

Then, the compound of the formula F is reduced to a compound of the formula B:

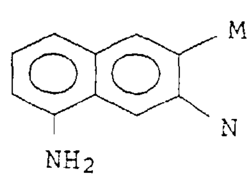

(B)

wherein M and N individually represent a hydroxyl or $C_1$ - $C_5$ alkoxy group, or M and N simultaneously represent a hydrogen atom or together form a methylenedioxy group. The 5-amino naphthalene, a compound of the formula B, is a known compound and is commercially available (Merck Index, 10, 6250).

The naphthylamine of formula B is condensed with 2-(protected hydroxy)-3-methoxy-6-halogenobenzaldehyde under dehydration conditions to form a condensation product which is a Schiff's base. Thereafter, the Schiff's base is selectively reduced so as to obtain the compound of formula C.

Next, a more detailed explanation will be made on the process mentioned above.

1. Synthesis of compounds of the general formula F

Compounds of the general formula F, wherein M and N together form a methylenedioxy group, can be produced by a process, wherein 2,3-dihydzoxy-5-nitronaphthalene is dissolved in a non-aqueous polar solvent such as dimethyl formamide or the like, and then reacted under heating at a temperature of from 60 to 110°C with a dihalogenomethane, preferably dichloromethane or dibromomethane, in the presence of a fluoride salt, preferably cesium fluoride or potassium fluoride as catalyst, to obtain 2,3-(methylenedioxy)-5-nitronaphthalene (compound 1).

In the case of compounds of the general formula F, wherein M and N individually represent a hydroxyl or $C_1$ - $C_5$ alkoxy group, such compounds may be prepared by a process, wherein 2,3-dihydroxy-5-nitronaphthalene is dissolved in a non-aqueous polar solvent such as dimethyl formamide or the like, and then alkylated by reacting with an alkyl halide such as methyl iodide, ethyl iodide, isopropyl bromide or the like, in the presence of an alkaline catalyst, for instance, $Li_2CO_3$ or $K_2CO_3$ at a temperature of from 50 to 100°C for 10 to 20 hours. When 1 mol of alkyl halide is reacted with the 2,3-dihydroxy-5-nitronaphthalene, two kinds of monoalkoxy compounds will be obtained. For the production of dialkoxy compounds, use should preferably be made of at least 2 mol of alkyl halides. If it is desired to produce a $C_1$ - $C_5$ alkoxy-substituted compound, wherein N and M are different from each other, then a process may be conducted, wherein a monoalkoxy compound, which has been isolated by means of a fractionation, is further reacted with an alkyl halide.

2. Synthesis of compounds having the general formula B

Compounds of the general formula B can be obtained by reducing the nitro group of compounds, having the general formula F, to an amino group. For carrying out this reaction, it is possible to use any reducing agents customarily employed in reducing a nitro group to an amino group. A preferred method comprises heating the reactant in ethanol in the presence of a 5 or 10% palladium/carbon catalyst under reflux.

2,3-methylenedioxy-5-aminonaphthalene may also be obtained by a known process described in J. Org. Chem., 53, 1708 (1988), although said known process has many steps, and produces the aimed compound in a low yield.

3. Synthesis of compounds having the general formula C

For this synthesis naphthylamine compounds of the general formula B are reacted with 2-(protected oxy)-3-methoxy-6-halogenobenzaldehyde (which can be produced by a known method described, for instance, in J. C. S. Perkin I, 1221 (1976) and J. Org. Chem., 53, 1708(1988)) by heating the reactants in toluene or benzene to a temperature of from 80 to 110°C for 1-3 hours and then by concentrating the reaction mixture in such a manner that the water, which has been produced as by-product by the condensation of the amino group with the aldehyde group, is effectively removed from the reaction system by an azeotropic distillation with toluene or benzene. It is preferred that, after the concentration, the resulting residue is admixed with fresh toluene or benzene, and then again subjected to a heating/concentration operation. By repeating 2-4 times the heating/concentration operation, it is possible to obtain a dehydrated condensate product (Schiff's base) in a substantially quantitative yield.

Then, the double bond of the condensed portion of the dehydrated condensate product is subjected to a reducing reaction to form the aimed compounds of the general formula C. In carrying out the reducing reaction, use may be made of any reducing agents which can reduce a CN double bond. Preferably, this reaction is performed in the presence of sodium cyanoborohydride or dimethylamine borone as reducing agent at a low reaction temperature of from -10 to 40°C.

Next, the detailed processes are explained.

A. Synthesis of compounds having the general formula D

A compound of the general formula C is subjected to a ring closure reaction (condensation reaction by elimination of hydrogen halide) in the presence of organic tin hydrides in an organic solvent. The preferable organic tin hydrides are tri-hydrocarbon ($C_1$-$C_6$)tin hydrides, e.g. triphenyltin hydride, tri-n-butyltin hydride, triethyltin hydride or trimethyltin hydride, or di-hydrocarbon ($C_1$-$C_6$)tin hydrides e.g. diphenyltin hydride or di-n-butyltin hydride. In general, tri-n-butyltin hydride is more preferable, and usually used. In carrying out this reaction, 1-6 equivalents, preferably 2-3 equivalents of organic tin hydride are dissolved in an organic solvent, preferably $C_6$-$C_{10}$ hydrocarbon solvent, for example toluene, xylene or benzene, and preferably admixed with a radical reaction initiator such as 2,2'-azobis(isobutyronitrile), 2,2'-azobis(2-methylbutyronitrile), 2,2'-azobis(2,4-dimethylvaleronitrile), benzoyl peroxide or the like. The reaction mixture is heated to a temperature of from 60 to 150°C, preferably 80 to 130°C for 2 minutes to 4 hours, preferably 5 minutes to 1 hour to complete the ring closure reaction. Thereafter, preferably without separating the

condensation product from reaction mixture, an oxidative aromatization reaction of the closed ring is carried out in the presence of an oxidizing agent at a temperature between 0 and 100°C preferably 10 and 40°C for 1 to 120 minutes, preferably 5 to 50 minutes, to obtain the compound of the general formula D.

Various oxidizing agents can be used for this reaction, and include, for example, manganese dioxide, lead tetraacetate, mercury acetate and dichloro dicyanobenzoquinone (DDQ), preferably active manganese dioxide.

By the above-mentioned reactions, the skeletons of benzo[c]phenanthridine will be formed.

As the halogen atom in the general formula C, use is generally made of a bromine atom. Referring to the protective groups W in the general formulas C and D, any kinds of protective groups for hydroxyl groups may be employed without any specific limitations, for example, acyl ($C_2$-$C_8$) groups such as acetyl and benzoyl, and $C_1$-$C_6$ alkyl carbonyl and $C_3$-$C_{10}$ hydrocarbon groups such as branched alkyl ($C_3$-$C_6$) groups and substituted or unsubstituted benzyl. The preferable protective groups are benzyl series protective groups such as substituted or unsubstituted benzyl groups, or branched alkyl ($C_3$-$C_5$) groups.

B. Synthesis of compounds having the general formula A'

A benzo[c]phenanthridine compound of the general formula D is subjected to an N-alkylation reaction, then the protective groups are removed from the compound, and thereafter an acid treatment is conducted to produce a benzo[c]phenanthridinium derivative of the general formula A'.

In effecting the N-alkylation, the compound of the general formula D and an alkylating agent may be dissolved in an organic solvent, for example, $C_6$-$C_{10}$ hydrocarbon solvent such as dry toluene, dry benzene, dry xylene or the like, or may be used in the absence of any solvents. These reactants are heated in the absence of catalysts or in the presence of salts such as alkalimetal halogenides or carbonates, preferably potassium bromide, anhydrous potassium carbonate, anhydrous sodium carbonate and the like.

The reaction temperature is usually 50° to 180°C, preferably 100 to 150°C. The reaction time is usually 1-24 hr, preferably 2 to 10 hr.

As the alkylating agents for the above reaction, use may be made of any of conventional agents customarily employed for N-alkylation reactions of pyridine rings. Examples of preferred alkylating agents include ($C_1$-$C_4$)alkyl sulfonates used for an alkylation such as $C_1$ - $C_5$ alkyl substituted benzensulfonates (for example, $C_1$ - $C_5$ alkyl p-toluenesulfonates, and more highly reactive agents such as $C_1$ - $C_5$ alkyl 2,4-dinitro-benzenesulfonates and $C_1$ - $C_5$ alkyl 2-nitrobenzenesulfonates) or $C_1$ - $C_5$ alkyl trihalogenomethane sulfonates. The alkylating agents are, for example, methyl p-toluensulfonate, ethyl 2,4-dinitrobenzenesulfonate, methyl 2-nitrobenzenesulfonate, n-propyl 2-nitrobenzenesulfonate and methylirifluoromethane sulfonate.

The protective group removal operation is carried out in consideration of the kinds of the protective groups to be removed. For instance, in the case of benzyl series protective groups or isopropyl group, such groups may be removed by heating at 60° to 150°C, preferably 80 to 120°C under acidic conditions, e.g. in the presence of concentrated hydrochloric acid. The reaction time is usually 0.1 to 10 hr preferably 0.5 to 3 hr.

After the protective groups have been removed from the compound, an acid treatment of the compound is carried out, for instance, in a manner, wherein the compound is dissolved in a small amount of a polar solvent such as methanol or the like, and then admixed with an acid, for example, hydrochloric acid, sulfuric acid or p-toluenesulfonic acid diluted with water. The pH of the solution must be below pH 4. An amount of an acid is usually about 1 to 3 mols per 1 mol of the compound. Furthermore, an organic solvent such as acetone, which is highly miscible with water, is added to the reaction solution to form salts as precipitates, and the reaction solution is dried, so that the compound of the general formula A' is obtained as yellow powder.

If sulfuric acid is used in a molar amount 1.0-2.5 times larger than the quantitative amount, then a hydrogen sulfate salt (wherein $X^-$ is $HSO_4^-$) will be formed. If sulfuric acid is employed in a half amount, then a normal type sulfate salt (wherein $X^-$ is 1/2 $SO_4^{2-}$) will be formed (not contained in the invention).

The compounds according to the invention have characteristic chemical properties as explained below. When the benzo[c]phenanthridinium derivatives of the general formula A is treated with a base, then compound will release 1 equivalent of the acid from the molecule. Therefore, it is considered that the compound may also have a form with an intramolecular zwitterion structure represented by the following formula E:

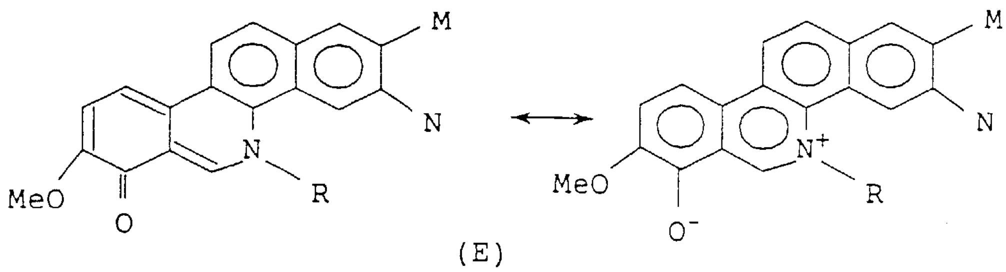

(E)

wherein M and N individually represent a hydroxyl or $C_1$ - $C_5$ alkyl group, or M and N simultaneously represent a hydrogen atom or together form a methylenedioxy group; and R represents a $C_1$ - $C_5$ alkyl group.

Alternatively, the compounds of the general formula E may also be treated with acids to convert them into the compounds of the general formula A'. The quaternary salt structure represented by the general formula A' is present in the solution having below pH about 4, while the intramolecular salt structure represented by the general formula E is present in the solution having higher than pH about 4. Therefore, the compound of formula A' can be obtained when the compound is precipitated from the solution of which the pH is below about 4.

As for the stability of the salt type compounds according to the invention, the acidic salts (i.e. hydrogen salts: $X^-$ represents hydrogen acid residue), which still contain protons (hydrogen atoms), have a resistance to foreign basic materials. Therefore, the acidic salts according to the invention are more stable than the normal salts containing no protons.

For example, even in the case of 2,3-(methylenedioxy)-5-methyl-7-hydroxy-8-methoxy-benzo[c]phenanthridinium chloride (not included in the invention) which seems to have relatively good properties (stability), there is a problem that, when it is stored at room temperature for a long period of time, it will be gradually decomposed. This compound is a powdery material having a yellowish color immediately after the production thereof. It gradually changes its color into brown. After 3 months from the production thereof, the compound has a dark blackish brown color and also has properties different from those initially observed. This is because a decomposition product, which is difficultly soluble in water, has been formed during the storage of the compound. So, it is not considered that the compound has properties suitable for formulating into a phamaceutical preparation.

2,3-(methylenedioxy)-5-methyl-7-hydroxy-8-ethoxy-benzo[c]phenanthridinium hydrogen sulfate (compound A-0), wherein the acid residue $X^-$ is a hydrogen sulfate ion $HSO_4^-$, is a compound according to the invention, and can be obtained as yellow powder. When this compound is stored at room temperature for 1 month, there is no change in its color, and therefore the compound is virtually different in this point from the above-mentioned chloride compound, wherein the acid residue $X^-$ is a chloride ion $Cl^-$. Furthermore, there are observed no color change and no decomposition in the case of the compound according to the invention having a form of yellow powder, even after it has been stored for 3 months from the production thereof. A 0.5 μM aqueous solution of 2,3-(methylenedioxy)-5-ethyl-7-hydroxy-8-methoxy-benzo[c]phenanthridinium chloride, wherein the acid residue $X^-$ is a chloride ion $Cl^-$, has a pH of 4.3. On the other hand, a 0.5 μM aqueous solution of 2,3-(methylenedioxy)-5-methyl-7-hydroxy-8-methoxy-benzo[c]phenanthridinium hydrogen sulfate according to the invention, wherein the acid residue $X^-$ is a hydrogen sulfate ion $HSO_4^-$, has a pH of 3.9. Thus, the hydrogen sulfate salt compound has a lower pH, and therefore it is recognized that said compound has a higher resistance to foreign basic materials and is more stable accordingly. There are no disadvantages in the pharmaceutical activities of such compounds when they have the form of hydrogen salts such as hydrogen sulfate salts. Therefore, the hydrogen salt compounds are practically useful as medicines in view of the enhanced stability thereof.

Next, representative examples of benzo[c]phenanthridinium derivatives of the general formula A' are shown in Table 1.

Table 1

| Compound No. | Compound |
|---|---|
| A-0 | 2,3-(Methylenedioxy)-5-methyl-7-hydroxy-8-methoxybenzo[c]phenanthridinium salt |
| A-1 | 2,3-(methylenedioxy)-5-ethyl-7-hydroxy-8-methoxybenzo[c]phenanthridinium salt |
| A-2 | 2,3-(Methylenedioxy)-5-(n-propyl)-7-hydroxy-8-methoxy-benzo[c]phenanthridinium salt |
| A-3 | 5-Ethyl-7-hydroxy-8-methoxy-benzo[c]phenan-thridinium salt |
| A-4 | 5-Methyl-7-hydroxy-8-methoxy-benzo[c]phenan-thridinium salt |

Representative examples of the intermediate compounds, having the general formula D, are shown in Table 2.

Table 2

| Compound No. | Compound |
|---|---|
| D-1 | 2,3-(Methylenedioxy)-7-benzyloxy-8-methoxy-benzo[c]phenanthridine |
| D-2 | 2,3-(Methylenedioxy)-7-isopropoxy-8-methoxy-benzo[c]phenanthridine |
| D-3 | 2,8-Dimethoxy-3-isopropoxy-7-benzyloxy-benzo[c]phenanthridine |
| D-4 | 2-Isopropoxy-3,8-dimethoxy-7-benzyloxy-benzo[c]phenanthridine |
| D-6 | 2,3-Diisopropoxy-7-benzyloxy-8-methoxy-benzo[c]phenanthridine |
| D-7 | 7-Benzyloxy-8-methoxy-benzo[c]phenanthridine |

Representative examples of the intermediate compounds, having the general formula C, are shown in Table 3.

Table 3

| Compound No. | Compound |
|---|---|
| C-1 | N-[(2'-benzyloxy)-3'-methoxy-6'-bromobenzyl]-6,7-(methylenedioxy)-1-naphthylamine |
| C-3 | N-[(2'-benzyloxy)-3'-methoxy-6'-bromobenzyl]-6-methoxy-7-isopropoxy-1-naphthylamine |
| C-4 | N-[(2'-benzyloxy)-3'-methoxy-6'-bromobenzyl]-6-isopropoxy-7-methoxy-1-naphthylamine |
| C-6 | N-[(2'-benzyloxy)-3'-methoxy-6'-bromobenzyl]-6,7-diisopropoxy-1-naphthylamine |
| C-7 | N-[(2'-benzyloxy)-3'-methoxy-6'-bromobenzyl]-1-naphthylamine |

Test

Now, the Pharmaceutical Test Examples are shown. The benzo[c]phenanthridinium derivatives according to the invention have a growth inhibition activity against various cultured tumor cells as shown below. Furthermore, the derivatives also have an activity for inhibiting a blood platelet aggregation caused by platelet activation factors.

1. Growth inhibition activity against tumor cells

Various tumor cells were cultured at 37°C for 24 hours in 5% gaseous carbon dioxide, and then subjected to an action of a test drug for 2-4 days. After that, the cells were stained with 0.05% Methylene Blue. The pigment were extracted from the stained cells. The growth inhibition rate of the cells was determined based on absorbance of the extracted pigment at 660 nm, and the 50% growth inhibitory concentration ($IC_{50}$) was calculated. The test results are shown in Table 4.

Table 4

$IC_{50}$ (μg/ml)

| Compound No. | A-0 ($X^-=HSO_4^-$) | A-1 ($X^-=HSO_4^-$) | A-2 ($X^-=HSO_4^-$) | A-3 ($X^-=HSO_4^-$) | A-4 ($X^-=HSO_4^-$) |
|---|---|---|---|---|---|
| HeLa $S_3$ | 0.15 | 0.177 | n.t. | 0.69 | 0.26 |
| MMI | 0.12 | 0.165 | 0.273 | n.t. | 0.262 |
| SHIN-3 | 0.192 | 0.170 | 0.414 | n.t. | 0.155 |
| N-231 | 0.036 | 0.040 | 0.085 | n.t. | 0.062 |
| Lu-130 | 0.055 | 0.250 | 0.108 | n.t. | 0.051 |
| Nakajima | n.t. | 0.426 | 0.777 | n.t. | n.t. |

In this table, the abbreviated expressions have the following tumor cells: HeLa S3 = carcinoma colli; MMI = ovarian cancer; SHIN-3 = ovarian cancer; N-231 = small cell lung cancer; Lu-130 = small cell lung cancer; Nakajima = gastric cancer; and the expression "n.t." represents "not tested". These tests were conducted according to a simultaneous comparison test method, except the case of HeLa $S_3$.

2. Inhibition activity on platelet aggregation

Platelet-rich plasma (PRP) was collected from Japanese albino rabbits (weighing 3-4 kg). As a platelet aggregation inducer, platelet activation factor (PAF) was used in a final concentration of $10^{-7}$ M. A test drug was added to PRP. After incubation for a definite period of time, PAF was added to cause a platelet aggregation reaction. The reaction was terminated with EDTA. After a centrifugation, the supernatant was removed to obtain platelet precipitates. Distilled water was added to the platelet precipitates, whereby the serotonin remained in the platelet was reacted with an orthophthalaldehyde reagent to form a serotonin-orthophthaladehyde condensate. The condensate was measured at an excited wavelength of 360 nm and at a measurement wavelength of 475 nm. Anti-PAF activity of the test drug

was determined by the following equation.

$$\text{Serotonin release inhibition rate (\%)} = \frac{\text{(Test drug + PAF) value - PAF value}}{\text{Blank value - PAF value}} \times 100$$

In the equation, the expression "value" stands for "serotonin value".

The test results of several compounds are shown in Table 5.

Table 5

| Test drug (Compound No.) | Concentration (μg/ml) | Inhibition rate (%) |
|---|---|---|
| A-1 ($X^-$=$HSO_4^-$) | 100 | 96.5 |
| | 50 | 74.1 |
| | 25 | 49.6 |
| | 12.5 | 25.2 |
| A-4 ($X^-$=$HSO_4^-$) | 100 | 46.4 |
| | 50 | 26.5 |
| | 25 | 10.9 |
| Physiological saline | - | 0.0 |

As is clear from Table 5, the test drugs showed a potent inhibition activity on platelet aggregation.

3. Acute toxicity

In the acute toxicity test, the compounds according to the invention were intravenously administered to female $CDF_1$ mice of 6 weeks age. The mice survived in a dose of 100 mg/kg without showing any lethal toxicity.

When the benzo[c]phenanthridinium derivatives of the general formula A' according to the invention are used as drugs, then these compounds may be formulated into pharmaceutical preparations, and such preparations may be applied in various conventional manners. That is, the preparations may be applied parenterally, orally, intrarectally, etc. The preparations may take the form of an injection, a powder, a granulate, a tablet, a suppository, etc. In preparing the pharmaceutical compositions, a variety of auxiliary agents for drugs, namely, carrier and other aids, for example, a stabilizer, an antiseptic, a pain killer, an emulsifying agent, etc. may be used, if necessary and desired.

In the pharmaceutical compositions, the content of the benzo[c]phenanthridinium derivatives, having the general formula A', may be varied over a wide range depending upon the form of preparations. Generally, the compositions may contain the derivatives of the general formula A' in an amount of from 0.01 to 100% by weight, preferably from 0.1 to 50% by weight. The balance is a carrier and other auxiliary agents used for conventional pharmaceutical compositions.

A dose of the benzo[c]phenanthridinium derivatives, having the general formula A, varies depending on conditions of patients, but generally is approximately 50 to 500 mg per day for adult.

From the test data shown above, it can be said that the benzo[c]phenanthridinium derivatives of the general formula A' according to the invention have not only an antitumor activity against various tumor cells, but also an inhibition activity on platelet aggregation, the latter activity being considered to have a relation with an activity for inhibiting a metastasis of cancer. So, it is expected that the compounds according to the invention may be effectively used as drugs for the treatment of cancer. When the compounds according to the invention are used in the form of salts, it is advantageous to use hydrogen salts of the compounds for the reason that the hydrogen salts of the compounds serve to enhance the stability of pharmaceutical preparations containing the compounds.

Now, a detailed explanation will be made about the production of several representative benzo[c]phenanthridinium derivatives having the general formula A', by the Examples shown below. However, it should be noted that the scope of the invention is not restricted only to these Examples.

**Reference Example 1**

Synthesis of 2,3-(methylenedioxy)-5-nitronaphthalene (compound 1)

19.3 g (0.094 mol) of 2,3-(dihydroxy)-5-nitronaphthalene as the starting material and 71.5 g (0.47 mol) of cesium fluoride were added to 290 ml of dry dimethyl formamide. The resulting mixture was stirred to form a solution.

The solution was admixed with 6.63 ml (0.103 mol) of dichloromethane, and heated to 110°C. After that, the operation of adding the same amount of dichloromethane was repeated four times at an interval of 1 hour, and then the reaction mixture was heated for 2 hours to complete the reaction. After cooling, the reaction mixture was diluted with water, and extracted with diethyl ether. The extract solution was dried over anhydrous sodium sulfate, filtered, and concentrated. The resultant residue was recrystallized from ethyl acetate, so that 10.7 g of the first crystals and 1.82 g of the second crystals were obtained. Furthermore, the mother liquor was concentrated and refined by a silica gel column chromatography, wherein use was made, as eluent, of a petroleum ether-ethyl acetate mixture (9:1), whereby 2.00 g of the crystalline material was obtained. The total amount of compound 1 formed was 14.55 g (yield: 71%).
Compound 1: yellow powder.

$^1$H-NMR(200 MHz), DMSO-$d_6$, ppm:

6.26 (s, 2H), 7.52 (t, J=8.0Hz, 1H), 7.58 (s, 1H), 7.74 (s, 1H), 8.55 (br d, J=8.0Hz, 2H).

**Reference Example 2**

Synthesis of 2,3-(methylenedioxy)-5-naphthylamine (compound 2)

4.687 g (0.0216 mol) of compound 1 were dissolved in 200 ml of ethanol. The resulting solution was admixed with 10 ml of hydrazine hydrate and 1 g of a 5% palladium/carbon catalyst, and heated under reflex for 80 minutes. After cooling, the palladium/carbon catalyst was filtered off, and the filtrate was concentrated. The crude crystalline material thus formed was refined by a silica gel column chromatography, wherein use was made, as eluant, of a petroleum ether-ethyl acetate mixture (1:2), so that 3.566 g of compound 2 was obtained (yield: 88%).
Compound 2: light yellowish brown powder.

$^1$H-NMR(200 MHz), DMSO-$d_6$, ppm:

6.07 (s, 2H), 5.44 (br s, 2H), 6.54 (dd, J=7.2 & 1.5Hz, 1H), 6.92 (br d, J=8.0Hz, 1H), 7.02 (dd, J=8.0 & 7.2Hz, 1H), 7.12 (s, 1H), 7.47 (s, 1H).

**Reference Example 3**

Synthesis of N-[(2'-benzyloxy)-3'-methoxy-6'-bromobenzyl]-6,7-(methylenedioxy)-1-naphthylamine (compound C-1)

3.548 g (18.95 mmol) of compound 2 and 6.304 g (19.63 mmol) of 2-(benzyloxy)-3-methoxy-6-bromobenzaldehyde were dissolved in 100 ml of toluene. The resulting solution was heated to 110°C for 1 hour, and then concentrated under reduced pressure in a rotary evaporator. The residue thus obtained was admixed with 80 ml of fresh toluene, and the resulting mixture was heated for 1 hour, and then concentrated under reduced pressure in the same manner as above. The resultant residue (Schiff's base) was admixed with 50 ml of toluene, and cooled in an ice water bath. Then, a solution of 1.31 g (20.85 mmol) of sodium cyanoborohydride in 50 ml of methanol was dropwise added to the cooled solution under stirring over a period of 45 minutes. After 1 hour, the reaction mixture was admixed with water, and extracted with chloroform. The organic layer was dried over anhydrous sodium sulfate, then filtered and thereafter concentrated. The resulting residue was dissolved in a small amount of chloroform, and then admixed portionwise with the same quantity of petroleum ether to precipitate a crystalline material, which was then separated by filtration, and dried, so that 5.674 g of compound C-1 were obtained. The mother liquor was concentrated, and refined by a silica gel column chromatography, wherein use was made, as eluant, of a petroleum ether-chloroform-ethyl acetate mixture (16:4:1), so that 1.415 g of compound C-1 was obtained. The total amount of compound C-1 formed was 7.089 g (yield: 76%).
Compound C-1: white powder.

$^1$H-NMR(200 MHz), $CDCl_3$, (ppm):

3.90 (s, 3H), 4.37 (br, 1H), 4.48 (s, 2H), 5.04 (s, 2H), 5.99 (s, 2H), 6.74 (dd, J=7.4 & 1.2Hz, 1H), 6.82 (d, J=8.8Hz, 1H), 7.05 (s, 1H), 7.07 (s, 1H), 7.11 (br d, J=8.0Hz, 1H), 7.33 (d, J=8.8Hz, 1H), 7.18-7.37 (m, 6H).

IR(KBr), ($cm^{-1}$):

3396, 3096, 3064, 3038, 2998, 2944, 2906, 2845, 1621, 1603, 1573, 1538(s), 1501, 1464(vs), 1435, 1400, 1393, 1371, 1316, 1285(s), 1271(s), 1251(s), 1218, 1202, 1194, 1173, 1144, 1131, 1099, 1072, 1040(s), 993, 960, 948, 916, 907, 862, 831, 795, 778, 757, 739, 687.

**Reference Example 4**

Synthesis of N-[(2'-benzyloxy)-3'-methoxy-6'-bromobenzyl]-6-methoxy-7-isopropoxy-1-naphthylamine (compound C-3)

463 mg of 2-methoxy-3-isopropoxy-5-naphthylamine and 707 mg of 2-(benzyloxy)-3-methoxy-6-bromobenzaldehyde were subjected to a reaction operation in a manner similar to that shown in Example 3. 970 mg of compound C-3 was obtained in a yield of 90%.
Compound C-3: white powder.
$^1$H-NMR(200 MHz), $CDCl_3$, (ppm):
1.36 (d, J=6.0Hz, 6H), 3.88 (s, 3H), 3.94 (s, 3H), 4.53 (s, 2H), 4.57 (septet, J=6.0Hz, 1H), 4.58 (br, 1H), 5.03 (s, 2H), 6.75 (dd, J=7.5 & 1.0Hz, 1H), 6.80 (d, J=9.0Hz, 1H), 7.08 (s, 1H), 7.09 (s, 1H), 7.13 (br d, J=7.5Hz, 1H), 7.22 (t, J=7.5Hz, 1H), 7.24-7.31 (m, 3H), 7.32 (d, J=9.0Hz, 1H), 7.34-7.41 (m, 2H).

**Reference Example 5**

Synthesis of N-[(2'-benzyloxy)-3'-methoxy-6'-bromobenzyl]-6-isopropoxy-7-methoxy-1-nahtylamine (compound C-4)

463 mg of 2-isopropoxy-7-methoxy-5-naphthylamine and 481 mg of 2-(benzyloxy)-3-methoxy-6-bromobenzaldehyde were subjected to a reaction operation in a manner similar to that of Example 3 to obtain 800 mg of compound C-4 (yield: 99%).
Compound C-4: white powder.
$^1$H-NMR(200 MHz), $CDCl_3$, (ppm):
1.43 (d, J=6.0Hz, 6H), 3.84 (s, 3H), 3.89 (s, 3H), 4.54 (s, 2H), 4.60 (br, 1F), 4.69 (septet, J=6.0Hz, 1H), 5.04 (s, 2H), 6.76 (dd, J=7.5 & 1.0Hz, 1H), 6.81 (d, J=9.0Hz, 1H), 6.97 (s, 1H), 7.105 (s, 1H), 7.113 (br d, J=7.5Hz, 1H), 7.22 (t, J=7.5Hz, 1h), 7.24-7.30 (m, 3H), 7.33 (d, J=9.0Hz, 1H), 7.32-7.41 (m, 2H).

**Reference Example 6**

Synthesis of N-[(2'-benzyloxy)-3'-methoxy-6'-bromobenzyl]-6,7-diisopropoxy-1-naphthylamine (compound C-6)

406 mg of 2,3-diisopropoxy-5-naphthylamine and 707 mg of 2-(benzyloxy)-3-methoxy-6-bromobenzaldehyde were subjected to a reaction operation in a manner similar to that of Example 3 to obtain 756 mg of compound C-6 (yield: 89%).
Compound C-6: white powder.
$^1$H-NMR(200 MHz), $CDCl_3$, (ppm):
1.32 (d, J=6.1Hz, 6H), 1.39 (d, J=6.1Hz, 6H), 3.88 (s, 3H), 4.49 (septet, J=6.1Hz, 1H), 4.51 (s, 2H), 4.55 (br, 1H), 4.61 (septet, J=6.1Hz, 1H), 5.03 (s, 2H), 6.72 (dd, J=7.5 & 1.1Hz, 1H), 6.80 (d, J=8.8Hz, 1H), 7.08-7.40 (m, 10H).
IR(KBr), ($cm^{-1}$):
3420, 2978, 2936, 1626, 1595, 1581, 1522, 1468(s), 1437, 1383, 1334, 1277, 1253(s), 1215, 1182, 1159, 1139, 1112, 1079, 1012, 982, 954, 925, 860, 840, 800, 773, 735, 695.

**Reference Example 7**

Synthesis of N-[(2'-benzyloxy)-3'-methoxy-6'-bromobenzyl]-1-naphthylamine (compound C-7)

1.718 g of 5-naphthylamine and 4.239 g of 2-(benzyloxy)-3-methoxy-6-bromobenzaldehyde were subjected to a reaction operation in a manner similar to that of Example 3 to obtain 3.71 g of compound C-7 (yield: 62.7%).
Compound C-7: white powder.
$^1$H-NMR(200 MHz), $CDCl_3$, (ppm):
3.90 (s, 3H), 4.52 (s, 2H), 4.80 (br, 1H), 5.06 (s, 2H), 6.80-6.85 (m, 2H), 7.22-7.47 (m, 10H), 7.68-7.81 (m, 2H)
IR(KBr), ($cm^{-1}$):
3999(s), 3070, 2944, 2856, 1626, 1581(s), 1531, 1481, 1467(s), 1432, 1410, 1372, 1355, 1336, 1296, 1272 (s), 1227, 1215, 1200, 1178, 1144, 1109, 1088, 1073(s), 983, 959, 911, 865, 795, 783, 764(s), 741, 690.

**Reference Example 8**

Synthesis of 2,3-(methylenedioxy)-7-benzyloxy-8-methoxybenzo[c]phenanthridine (compound D-1)

1.968 g (4 mmol) of compound C-1 and 2.91 g (10 mmol) of tributyltin hydride were dissolved in 100 ml of toluene, and the resulting solution was heated to 110°C. To this solution, 0.8 g of 2,2'-azobis(isobutyronitrile) were added. After 30 minutes, the reaction solution was cooled to room temperature, then admixed with 2 g of active manganese dioxide, and stirred for 20 minutes. Next, the manganese compound was separated off by filtration, and the filtrate was concentrated under reduced pressure. The residue thus obtained was passed through a silica gel column, wherein use was made, as eluant, of a chloroform-petroleum ether mixture (3:2). The main fractions were collected and concentrated to obtain a crude crystalline material, which was then admixed with 25 ml of ethyl acetate, and heated under shaking. This solution was gradually admixed with 200 ml of hexane, and a crystalline material thus formed was separated by filtration, washed in hexane and dried to give 1.018 g of compound D-1. Furthermore, the filtrate was concentrated, and refined by a silica gel column chromatography, wherein use was made, as eluant, of a petroleum ether-chloroform-ethyl acetate mixture (16:4:1), so that 0.183 g of compound D-1 was further obtained. The total amount of compound D-1 produced was 1.201 g (yield: 73%).
Compound D-1: light yellowish white powder.

$^1$H-NMR(200 MHz), $CDCl_3$, (ppm):

4.07 (s, 3H), 5.32 (s, 2H), 6.13 (s, 2H), 7.26 (s, 1H), 7.34-7.46 (m, 3H), 7.58 (dd, J=8.0 & 1.6Hz, 2H), 7.61 (d, J=9.0Hz, 1H), 7.84 (d, J=9.0Hz, 1H), 8.34 (d, J=9.0Hz, 1H), 8.36 (d, J=9.0Hz, 1H), 8.69 (s, 1H), 9.75 (s, 1H).

$^{13}$C-NMR(50 MHz), $CDCl_3$, (ppm):

50.03(q), 76.21(t), 101.62(t), 102.46(d), 104.68(d), 118.57(d), 118.69(d), 119.02(d), 120.27(s), 122.48(s), 127.40(d), 128.43(s), 128.67(d), 128.87(d)x2, 128.96(d)x2, 129.51(s), 130.11(s), 137.59(s), 140.37(s), 144.38(s), 147.22(d), 148.71(s), 148.89(s), 149.91(s).

IR(KBr), ($cm^{-1}$) :

3032, 3004, 2964, 2944, 2910, 2875, 2840, 1640, 1616, 1595, 1578, 1533, 1495, 1460(vs), 1440(s), 1394, 1378, 1358, 1324, 1284(s), 1277(s), 1251(s), 1223, 1202(s), 1169, 1136, 1112, 1081(s), 1040(s), 991, 968, 958, 949, 920, 904, 875, 850, 845, 830, 795, 759, 750, 697, 686, 667.

**Reference Example 9**

Synthesis of 2,8-dimethoxy-3-isopropoxy-7-benzyloxybenzo[c]phenantridine (compound D-3)

886 mg of compound C-3 were subjected to a reaction operation in a manner similar to that of Example 8. 512 mg of compound D-3 was obtained (yield: 68%).
Compound D-3: light yellowish white powder.

$^1$H-NMR(200 MHz), $CDCl_3$, (ppm):

1.54 (d, J=6.1Hz, 6h), 4.04 (s, 3H), 4.06 (s, 3H), 5.05 (septet, J=6.1Hz, 1H), 5.31 (s, 2H), 7.28 (s, 1H), 7.34-7.47 (m, 3H), 7.55-7.61 (m, 2H), 7.59 (d, J=9.0Hz, 1H), 7.86 (d, J=9.0Hz, 1H), 8.33 (d, J=9.0Hz, 1H), 8.35 (d, J=9.0Hz, 1H), 8.77 (s, 1H), 9.77 (s, 1H)

IR(KBr), ($cm^{-1}$):

2976, 2938, 2838, 1618, 1576, 1525, 1509, 1479, 1461, 1434, 1411, 1389, 1372, 1351, 1324, 1282, 1265 (s), 1218, 1203, 1168, 1143, 1115, 1080, 1068, 1026, 981, 953, 929, 915, 880, 856, 842, 830, 797, 759, 747, 698, 685

**Reference Example 10**

Synthesis of 2-isopropoxy-3,8-dimethoxy-7-benzyloxybenzo[c]phenantridine (compound D-4)

720 mg of compound C-4 were subjected to a reaction operation in a manner similar to that of Example 8. 320 mg of compound D-4 was obtained (yield: 53%).
Compound D-4: light yellowish white powder.

$^1$H-NMR(200 MHz), $CDCl_3$, (ppm):

1.51 (d, J=6.0Hz, 6H), 4.07 (s, 3H), 4.17 (s, 3H), 4.80 (septet, J=6.0Hz, 1H), 5.32 (s, 2H), 7.32 (s, 1H), 7.34-7.47 (m, 3H), 7.55-7.62 (m, 2H), 7.62 (d, J=9.0Hz, 1H), 7.86 (d, J=9.0Hz, 1H), 8.36 (d, J=9.0Hz, 1H), 8.39 (d, J=9.0Hz, 1H), 8.72 (s, 1H), 9.79 (s, 1H).

IR(KBr), ($cm^{-1}$):

2976, 2938, 2840, 1618, 1578, 1527, 1509, 1478, 1456, 1435, 1409, 1389, 1375, 1355, 1325, 1267(s), 1220, 1191, 1177, 1163, 1140, 1112, 1083, 1067, 1021, 995, 973, 942, 911, 872, 849, 830, 804, 772, 759, 749, 698.

**Reference Example 11**

Synthesis of 2,3-diisopropoxy-7-benzyloxy-8-methoxybenzo[c]phenantridine (compound D-6)

692 mg of compound C-6 were subjected to a reaction operation in a manner similar to that of Example 8. 379 mg of compound D-6 was obtained (yield: 64%).
Compound D-6: light yellowish white powder.
$^1$H-NMR (200 MHz), $CDCl_3$, (ppm):
1.45 (d, J=6.1Hz, 6H), 1.49 (d, J=6.1Hz, 6H), 4.04 (s, 3H), 4.69 (septet, J=6.1Hz, 1H), 4.92 (septet, J=6.0Hz, 1H), 5.31 (s, 2H), 7.34-7.46 (m, 3H), 7.36 (s, 1H), 7.55-7.62 (m, 2H), 7.58 (d, J=9.0Hz, 1H), 7.83 (d, J=9.0Hz, 1H), 8.31 (d, J=9.0Hz, 1H), 8.34 (d, J=9.0Hz, 1H), 8.78 (s, 1H), 9.77 (s, 1H).
IR(KBr), ($cm^{-1}$):
2976, 2932, 1617, 1576, 1527, 1507, 1463, 1439, 1413, 1385, 1373, 1355, 1323, 1265(s), 1216, 1180, 1166, 1139, 1113, 1086, 1066, 998, 952, 932, 910, 876, 856, 831, 800, 743, 697.

**Reference Example 12**

Synthesis of 7-benzyloxy-8-methoxy-benzo[c]phenantridine (compound D-7)

3.71 g of compound C-7 were subjected to a reaction operation in a manner similar to that of Example 8. 1.387 g of compound D-7 was obtained (yield: 46%).
Compound D-7: light yellowish white powder.
$^1$H-NMR (200 MHz), $CDCl_3$, (ppm):
4.08 (s, 3H), 5.34 (s, 2H), 7.34-7.47 (m, 3H), 7.55-7.61 (m, 2H), 7.64 (d, J=9.0Hz, 1H), 7.61-7.71 (m, 1H), 7.71-7.81 (m, 1H), 7.97 (dd', J=8.0 & 1.3Hz, 1H), 8.00 (d, J=9.0Hz, 1H), 8.41 (d, J=9.0Hz, 1H), 8.48 (d, J=9.0Hz, 1H), 9.36 (dd, J=8.0 & 1.0Hz, 1H), 9.84 (s, 1H).
IR(KBr), ($cm^{-1}$):
3025, 2985, 2925, 2870, 2830, 1617, 1574, 1525, 1464, 1442, 1425, 1355, 1332, 1291(s), 1281(s), 1261, 1236, 1204, 1177, 1154, 1138, 1079(s), 1024, 986, 958, 934, 901, 868, 843, 834, 809(s), 816, 744, 721, 695.

The following Examples relate to the production of compounds having the general formula A'. These compounds may also be present in another form represented by the general formula E. So, the physical properties of the latter isomers are also given in the Examples, as far as such data were obtained.

**Example 1**

Synthesis of 2,3-(methylenedioxy)-5-methyl-7-hydroxy-8-methoxy-benzo[c]phenanthridinium hydrogen sulfate salt (compound A-0)

1.00 g (2.44 mmol) of compound D-1, 4.55 g (24.4 mmol) of methyl p-toluenesulfonate and 2.91 g (24.5 mmol) of KBr were sufficiently mixed with one another with the aid of a magnetic stirrer. The resulting mixture was then heated to 130°C for 5.5 hours, diluted with water, extracted with methylene chloride, dried over anhydrous sodium sulfate, filtered, and concentrated. The concentrated residual material thus obtained was again admixed with 4.55 g of methyl p-toluenesulfonate and 2.91 g of powdery KBr with the aid of a magnetic stirrer, and then heated to 130°C for 3 hours.

Next, the reaction mixture was admixed with 5 ml of acetic acid and 2.5 ml of concentrated hydrochloric acid, and heated to 100°C for 2 hours. After that, the reaction mixture was diluted with water, and neutralized by portionwise adding sodium hydrogen carbonate. The reaction mixture was extracted with methylene chloride, dried over anhydrous sodium sulfate, filtered and concentrated. Then the reaction mixture was subjected to a silica gel column chromatography, wherein use was made, as eluant, of a methylene chloride-methanol mixture (9:1). The fractions, which contained a pure compound E-0, were collected and concentrated.

The concentrated product, containing compound E-0, was gradually added to a dilute aqueous sulfuric acid solution containing 5 ml of a 0.5 M aqueous sulfuric acid solution, 25 ml of water and ice. The resulting mixture was concentrated, and then admixed with 5 ml of fresh water to dissolve a large portion of compound A-0 in the water. Thereafter, the aqueous solution thus obtained was gradually admixed with 200 ml of acetone to form a yellow precipitate, which was then separated by filtration, and dried in vacuum, whereby 750 mg of compound A-0 was obtained (yield: 71.2%).
Compound E-0: dark violet solid material
$^1$H-NMR(200 MHz), DMSO-$d_6$, (ppm):
3.76 (s, 3H), 4.41 (s, 3H), 6.25 (s, 2H), 7.04 (d, J=8.0Hz, 1H), 7.20 (d, J=8.0Hz, 1H), 7.55 (s, 1H), 7.90 (d,

J=9.0Hz, 1H), 7.98 (s, 1H), 8.27 (d, J=9.0Hz, 1H), 9.02 (s, 1H).

$^{13}C$-NMR(50 MHz), DMSO-$d_6$, (ppm):

48.65(q), 55.59(q), 100.04(d), 102.29(t), 103.22(d), 105.52(d), 118.54(s), 119.13(d), 119.36(s), 120.64(s), 125.41(s), 127.04(d), 128.30(d), 131.48(s), 131.63(s), 147.90(s), 148,09(s), 151.06(d), 151.21(s), 168.30(s)

IR(KBr), ($cm^{-1}$):

3050, 2964, 2910, 2834, 1625(s), 1584, 1559, 1538(s), 1511(s), 1501(s), 1474(s), 1459, 1417, 1372(s), 1336, 1287, 1256(vs), 1245(vs), 1207, 1180, 1145, 1122, 1114, 1098, 1042(s), 968, 940, 922, 891, 872, 864, 855, 828, 804, 790, 764, 745, 710, 680, 660.

Compound A-0: yellow powder ($X^-$=$HSO_4^-$)

$^1H$-NMR (200 MHz), $D_2O$, (ppm):

3.62 (s, 3H), 3.94 (br s, 3H), 5.95 (br s, 2H), 6.39 (s, 1H), 6.78 (s, 1H), 6.86 (d, J=9.OHz, 1H), 6.94 (d, J=9.0Hz, 1H), 7.02 (d, J=9.0Hz, 1H), 7.06 (d, J=9.0Hz, 1H), 8.56 (s, 1H)

$^{13}C$-NMR(50 MHz), $D_2O$, (ppm):

54.93(q), 58.96(q), 105.51(d), 106.16(t), 108.33(d), 115.55(d) & (s), 119.54(d), 121.05(s), 125.69(s), 125.81 (d), 127.67(s), 131.13(s), 133.47(d), 133.90(s), 146.94(s), 147.97(s), 150.79(s), 151.54(d) & (s)

IR(KBr), ($cm^{-1}$):

3476, 3040, 3010, 2950, 1628, 1603, 1585, 1549, 1493(s), 1478(s), 1466, 1447, 1415, 1373, 1353, 1338, 1297(s), 1279(s), 1260(s), 1218(s), 1191(s), 1156, 1112, 1096, 1053, 1038(s), 967, 919, 865, 847, 830, 778, 764, 751, 709.

**Example 2**

Synthesis of 2,3-(methylenedioxy)-5-ethyl-7-hydroxy-8-ethoxy-benzo[c]phenanthridinium hydrogen sulfate salt (compound A-1)

1.433 g (3.5 mmol) of 2,3-(methylenedioxy)-7-benzyloxy-8-methoxy-benzo[c]phenanthridine (compound D-1) was admixed with 0.906 ml (3.7 mmol) of ethyl trifluoromethanesulfonate and 10 ml of dry toluene, and heated to 100°C for 4.5 hours. Thereafter, the resultant mixture was admixed with 10 ml of acetic acid and 5 ml of concentrated hydrochloric acid, and heated to 100°C for 1 hour. After that, the reaction mixture was diluted by gradually adding the reaction mixture to ice water. The reaction mixture was then neutralized by portionwise adding sodium hydrogen carbonate. Thereafter, an extraction operation was effected with chloroform. The chloroform extract layer was dried over anhydrous sodium sulfate, filtered, and concentrated under reduced pressure. The residue thus obtained was refined by a silica gel chromatography, wherein use was made, as eluant, of a chloroform-methanol mixture (9:1). The main fractions were collected and concentrated to obtain a dark violet solid material, which was compound E-1 having a structure represented by the general formula E. Then, compound E-1 was dissolved in a small amount of methanol, and the resulting solution was acidified by gradually adding thereto a dilute aqueous sulfuric acid solution in an amount of from 1.0 to 2.0 mol per 1 mol of compound E-1. By this treatment, the color of the solution was changed into yellow or orange. Thereafter, the solution was concentrated under reduced pressure, and the concentrated aqueous solution was portionwise added with acetone under shaking to form a precipitate, which was separated by filtration and dried, so that compound A-1, wherein $X^-$ = $HSO_4^-$, was obtained as yellow powder in an amount of 1.171 g (yield: 75.1%).

Compound E-1: dark violet solid material.

$^1H$-NMR(200 MHz), $CDCl_3$ + $CD_3OD$ (1:1), (ppm):

1.65 (t, J=7.2Hz, 3H), 3.95 (s, 3H), 4.83 (q, J=7.2Hz, 2H), 6.19 (s, 2H), 7.30 (s, 1H), 7.30 (d, J=8.2Hz, 1H), 7.44 (d, J=8.2Hz, 1H), 7.56 (s, 1H), 7.81 (d, J=8.9Hz, 1H), 8.18 (d, J=8.9Hz, 1H), 9.36 (s, 1H)

$^{13}C$-NMR(50 MHz), $CDCl_3$ + $CD_3OD$ (1:1), (ppm):

16.65(q), 54.90(t), 56.36(q), 102.71(d), 103.14(t), 103.54(d), 106.56(d), 119.61(d), 119.77(s), 120.94(d), 121.47(s), 127.20(s), 128.10(s), 129.37(d), 131.33(s), 132.58(s), 149.29(s), 149.71(s), 150.84(d), 152.12(s), 168.45(s).

IR(KBr), ($cm^{-1}$):

2970, 2918, 1623(s), 1582, 1558, 1534(s), 1502(s), 1475(s), 1460(s), 1376(s), 1345, 1300, 1285, 1250 (vs), 1189, 1142, 1115, 1099, 1036(s), 941, 864, 794, 761, 736, 707, 673, 664.

Compound A-1: yellow powder ($X^-$ = $HSO_4^-$)

$^1H$-NMR(200 MHz), $D_2O$, (ppm):

1.64 (br t, J=6.3Hz, 3H), 3.63 (s, 3H), 4.27 (br m, 2H), 6.02 (br s, 2H), 6.45 (s, 1H), 6.55 (br s, 1H), 6.81 (d, J=9.0Hz, 1H), 6.95 (br, s, 2H), 7.05 (d, J=9.0Hz, 1H), 8.79 (s, 1H).

$^{13}C$-NMR(50 MHz), $D_2O$, (ppm):

19.32(q), 58.78(q), 59.43(t), 104.40(d), 105.92(t), 108.03(d), 115.24(d), 115.63(s), 119.17(d), 120.05(s), 125.49(s) & (d), 127.39(s), 130.66(s), 132.96(d), 133.33(s), 146.54(s), 147.55(s), 150.25(d), 150.59(s), 151.46(s).

IR(KBr), ($cm^{-1}$):

3364, 3040, 2970, 2930, 2888, 1622, 1602, 1550, 1491(s), 1474(s), 1453, 1412, 1383, 1348, 1334, 1300, 1279(s), 1260(s), 1223(s), 1217(s), 1158, 1128, 1115, 1103, 1080, 1042(s), 942, 925, 884, 825, 771.

**Example 15**

Synthesis of 2,3-(methylenedioxy)-5-(n-propyl)-7-hydroxy-8-methoxy-benzo[c]phenanthridinium hydrogen sulfate salt (compound A-2)

Compound D- 1 and n-propyl 2-nitrobenzene-sulfonate were subjected to a reaction operation in a manner similar to that of Example 14 to produce compound E-2, which was then treated with dilute sulfuric acid in the presence of methanol, whereby compound A-2, wherein $X^-$ = $HSO_4^-$, was obtained.
Compound A-2: yellow powder ($X^-$ = $HSO_4^-$)
$^1$H-NMR(200 MHz), $D_2O$, (ppm):
0.97 (br t, J=7.1Hz, 3H), 1.95 (br m, 2H), 3.79 (s, 3H), 4.22 (br m, 2H), 6.12 (br s, 2H), 6.55 (br s, 1H), 6.65 (s, 1H), 7.10 (d, J=9.0Hz, 1H), 7.16 (d, J=9.0Hz, 1H), 7.26 (d, J=9.0Hz, 1H), 7.30 (d, J=9.0Hz, 1H), 8.97 (s, 1H)
$^{13}$C-NMR(50 MHz) $D_2O$, (ppm)
l2.40(q) , 27.92(t), 59.22(q), 65.30(t), 104.49(d), 106.31(t), 108.62(d), 116.05(d), 116.30(s), 120.09(d), 120.79(s), 126.27(d), 126.40(s), 128.26(s), 131.31(s), 133.53(d), 134.08(s), 147.41(s), 148.36(s), 151.10(d), 151.27 (s), 152.02(s)
IR(KBr), ($cm^{-1}$):
3394, 3064, 2982, 1647, 1620, 1602, 1581, 1550, 1493(s), 1472(s), 1417, 1387, 1375, 1355, 1302(s), 1277 (s), 1257(s), 1215(s), 1172(s), 1155, 1139, 1117, 1105, 1082, 1062, 1039(s), 1001, 971, 934, 926, 886, 855(s), 829, 811, 790, 759, 707

**Example 4**

Synthesis of 5-ethyl-7-hydroxy-8-methoxy-benzo[c]phenanthridinium hydrogen sulfate salt (compound A-3)

183 mg (0.5 mmol) of 7-benzyloxy-8-methoxybenzo[c]phenanthridine (compound D-7) were admixed with 0.13 ml (1.0 mmol) of ethyl trifluoromethanesulfonate and 0.9 ml of dry toluene, and the resulting mixture was heated to 100°C for 5 hours. After that, the mixture was admixed with 4 ml of acetic acid and 1 ml of concentrated hydrochloric acid, and heated to 100°C for 1 hour. Thereafter, the reaction mixture was gradually added to ice water. The reaction mixture thus diluted was neutralized by portionwise adding thereto sodium hydrogen carbonate, and then extracted with chloroform. The chloroform extract layer was dried over anhydrous sodium sulfate, filtered, and concentrated under reduced pressure. The resultant residue was refined by a silica gel chromatography, wherein use was made, as eluant, of a chloroform-methanol mixture (9:1). The main fractions were collected and concentrated to obtain a dark violet solid material, which was compound E-7 having a structure represented by the general formula E. Next, compound E-7 was dissolved in a small amount of methanol, and the solution thus obtained was acidified by portionwise adding thereto a dilute aqueous solution of sulfuric acid in an amount of from 1.0 to 2.0 mol per 1 mol of the compound, so that the color of the solution of the compound was changed into yellow or orange. The aqueous solution of the compound was then concentrated under reduced pressure. Acetone was added portionwise to the concentrated solution under shaking to form a precipitate, which was separated by filtration, and dried, so that the aimed compound A-3, wherein $X^-$ = $HSO_4^-$, was obtained as yellow powder in an amount or 160 mg (yield: 79.7%).
Compound E-7: dark violet solid material.
$^1$H-NMR (200 MHz), DMSO-$d_6$, (ppm):
1.45 (t, J=7.1Hz, 3H), 3.78 (s, 3H), 4.82 (q, J=7.1Hz, 2H), 7.10 (d, J=8.0Hz, 1H), 7.21 (d, J=8.0Hz, 1H), 7.63-7.76 (m, 2H), 8.03 (d, J=8.8Hz, 1H), 8.08-8.14 (m, 1H), 8.32-8.41 (m, 1H), 8.40 (d, J=8.8Hz, 1H), 9.11 (s, 1H),
$^{13}$C-NMR(50 MHz), DMSO-$d_6$, (ppm):
15.96(q), 53.13(t), 55.23(q), 99.29(d), 117.77(d), 118.88(s), 120.57(d), 123.89(s), 124.56(d), 126.38(s), 126.54(s), 126.61(d), 126.68(f), 128.08(d), 128.86(d), 130.38(s), 133.47(s), 148.66(d), 151.53(s), 169.42(s).
IR(KBr), ($cm^{-1}$):
3039, 2986, 2938, 2832, 1628(s), 1614(s), 1557(s), 1530(s), 1510(s), 1495, 1474, 1456, 1423, 1386, 1377 (s), 1354, 1345, 1335, 1317, 1287, 1266(s), 1245(s), 1239(s), 1194, 1155, 1139, 1101(s), 1075, 1061, 1046, 977, 945, 923, 907, 871, 823, 798, 792, 774(s), 752, 691, 675.
Compound A-3: yellow powder ($X^-$ = $HSO_4^-$)
$^1$H-NMR(200 MHz), $D_2O$, (ppm):
1.72 (t, J=7.0Hz, 3H), 3.56 (s, 3H), 4.52 (br q, J=7.0Hz, 2H), 6.95 & 7.05 (AB, JAB=9.2Hz, each 1H), 7.21 (br s, 2H), 7.32-7.50 (m, 2H), 7.38 (br d, J=7.9Hz, 1H), 7.59 (br d, J=7.9Hz, 1H), 8.96 (s, 1H)

$^{13}$C-NMR(50 MHz) , $D_2O$, (ppm):
19.21(q), 58.82(q), 59.83(t), 115.65(d), 116.24(s), 120.59(d), 124.28(s), 125.66(d), 127.01(s) & (d), 127.52 (s), 131.05(d), 131.38(s) & (d), 132.25(d), 134.28(d), 135.72(s), 146.64(s), 147.90(s), 150.21(d)
IR(KBr), (cm$^{-1}$):
3419, 3052, 1614, 1583, 1540, 1497, 1468, 1446, 1431, 1384, 1361, 1341, 1302(s), 1277(s), 1216(s), 1164, 1150, 1143, 1130, 1086, 1059, 985, 921, 854, 821, 767, 695

**Example 5**

Synthesis of 5-methyl-7-hydroxy-8-methoxy-benzo[c]phenanthridinium hydrogen sulfate salt (compound A-4)

The starting material used here was compound E-8 prepared according to a method described in J. Org. Chem., 53, 1708-1713 (1988). Compound E-8 was subjected to a reaction operation to form compound A-4, in a manner similar to that of Example 2 directed to the conversion of compound E-1 into the corresponding hydrogen sulfate salt. Compound A-4 was obtained in a yield of 99.0%.
Compound A-4: yellow powder ($X^- = HSO_4^-$).
$^1$H-NMR(200 MHz), $D_2O$, (ppm):
3.42 (s, 3H), 4.08 (s, 3H), 6.79 & 6.85 (AB, JAB=9.1Hz, each 1H), 7.03 (br s, 2H), 7.16-7.24 (m, 1H), 7.24-7.34 (m, 2H), 7.63-7.73 (m, 1H), 8.61 (s, 1H).
$^{13}$C-NMR(50 MHz), $D_2O$, (ppm):
54.98(q), 58.81(q), 115.64(d), 115.87(s), 120.58(d), 124.93(s), 125.69(d), 126.84(s), 127.36(s), 127.98(d), 131.01(d), 131.60(d) & (s), 132.41(d), 134.66(d), 136.01(s), 146.82(s), 148.09(s), 151.29(d).
IR(KBr), (cm$^{-1}$):
3480, 3000, 2950, 1624, 1617, 1583, 1537, 1488, 1463, 1450, 1430, 1381, 1359, 1339, 1318, 1295(s), 1278(s), 1263, 1241, 1228(s), 1196, 1182, 1167, 1143, 1112(s), 1085, 1066, 982, 922, 914, 875, 853, 823(s), 783, 766, 751, 702, 692, 670.

**Example 6**

Pharmaceutical composition

1 g of 2,3-(methylenedioxy)-5-methyl-7-hydroxy-8-methoxy-benzo[c]phenanthridinium hydrogen sulfate salt (compound A-0), wherein $X^- = HSO_4^-$, 1 g of polysolvate and 1 g of Macrogol 400 were dissolved in 100 g of distilled water for injection, to form a solution. After filtering through a membrane filter, the solution was separately charged in ampules, and lyophilized in a conventional manner to give a preparation for injection containing 50 mg of compound A-0 per ampule.

**Example 7**

Pharmaceutical composition

1 g of 2,3-(methylenedioxy)-5-ethyl-7-hydroxy-8-methoxy-benzo[c]phenanthridinium hydrogen sulfate salt (compound A-1), wherein $X^- = HSO_4^-$, 1 g of polysolvate and 1 g of Macrogol 400 were dissolved in 100 a of distilled water for injection, to form a solution. After filtering through a membrane filter, the solution was separately charged in ampoules, and lyophilized in a conventional manner to give a preparation for injection containing 50 mg of compound A-1 per ampoule.

## Claims

**Claims for the following Contracting States : DE, FR, GB, IT, NL, SE**

**1.** A benzo[c] phenanthridinium derivative of the general formula A':

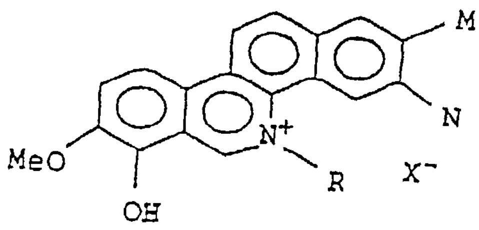

(A')

wherein M and N individually represent a hydroxyl or a $C_1$ - $C_5$ alkoxy group, or M and N simultaneously represent a hydrogen atom or together form a methylenedioxy group, $X^-$ represents $HSO_4$- or $H_2PO_4^-$, and R represents a $C_1$ - $C_5$ alkyl group.

2. The benzo [c] phenanthridinium derivative according to claim 1 wherein the $C_1$ - $C_5$ alkoxy group is a selected from the group consisting of the methoxy-, the ethoxy-, the n-propoxy- and the iso-propoxy-group.

3. The benzo [c] phenanthridinium derivative according to claim 1 wherein M and N together form a methylenedioxy group.

4. The benzo [c] phenanthridinium derivative according to claim 3 wherein the $C_1$ - $C_5$ alkyl group is selected from the group consisting of the methyl-, the ethyl-, the n-propyl- and the iso-propyl-group.

5. The benzo [c] phenanthridinium derivative of formula A' according to claim 1 being 2,3-(methylenedioxy)-5-methyl-7-hydroxy-8-methoxy-benzo [c] phenanthridinium hydrogen sulfate salt.

6. The benzo [c] phenanthridinium derivative of formula A' according to claim 1 being 2,3-(methylenedioxy)-5-ethyl-7-hydroxy-8-methoxy-benzo [c] phenanthridinium hydrogen sulfate salt.

7. The benzo [c] phenanthridinium derivative of formula A' according to claim 1 being 5-methyl-7-hydroxy-8-methoxy-benzo [c] phenanthridinium hydrogen sulfate salt.

8. A pharmaceutical composition for the use as an antitumor or anti-platelet agent comprising as effective ingredient a compound according to one of the previous claims and pharmaceutically acceptable carriers and/or additives.

**Claims for the following Contracting State : ES**

1. Process for preparing a benzo[c]phenanthridinium derivative of the general formula A'

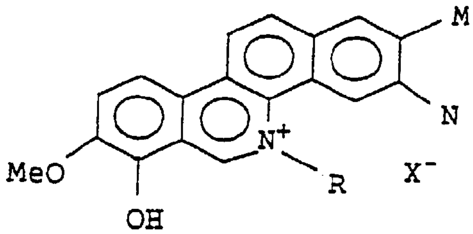

(A')

wherein M and N individually represent a hydroxyl or $C_1$ - $C_5$ alkoxy group, or M and N simultaneously represent a hydrogen atom or together from a methylenedioxy group; $X^-$ represents $HSO_4^-$ or $H_2PO_4^-$; and R represents a $C_1$ - $C_5$ alkyl group, wherein a compound of the general formula C:

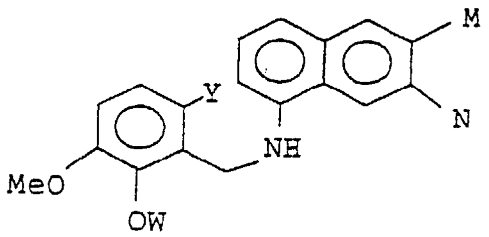

(C)

wherein M and N individually represent a hydroxyl or $C_1$ - $C_5$ alkoxy group, or M and N simultaneously represent a hydrogen atom or together form a methylenedioxy group; Y represents a halogen atom; and W represents a protective group, is subjected to a ring closure reaction in the presence of an organic tin compound, and then to an oxidative aromatization reaction to obtain a compound of the general formula D;

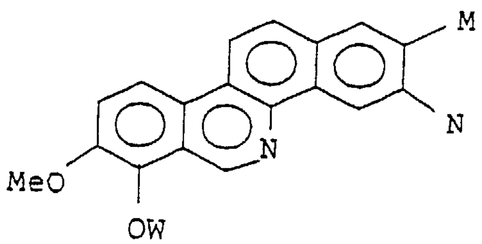

(D)

wherein M and N individually represent a hydroxyl or $C_1$ - $C_5$ alkoxy group, or M and N simultaneously represent a hydrogen atom or together form a methylenedioxy group; and W represents a protective group, and wherein the compound D thus formed is reacted with an N-alkylating agent to effect an N-alkylation of said compound; and wherein the N-alkylated compound thus formed is subjected to a protective group-removing operation and treated with $H_2SO_4$ or $H_3PO_4$ to form the aimed salt type product.

**2.** The process for preparing the benzo[c]phenanthridinium derivative according to claim 1 wherein $C_1$ - $C_5$ alkoxy group is a selected from the group consisting of the methoxy-, the ethoxy-, the n-propoxy and the iso-propoxy-group.

**3.** The process for preparing the benzo[c]phenanthridinium dervative according to claim 1 wherein M and N together form a methylenedioxy group.

**4.** The process for preparing the benzo[c]phenanthridinium derivative according to claim 3 wherein the $C_1$ - $C_5$ alkyl group is selected from the group consisting of the methyl-, the ethyl-, the n-propyl- and the iso-propyl-group.

**5.** The process for preparing the benzo[c]phenanthridinium derivative of formula A' according to claim 1 being 2,3-(methylenedioxy)-5-methyl-7-hydroxy-8-methoxybenzo[c]phenanthridinium hydrogen sulfate salt.

**6.** The process for preparing the benzo[c]phenanthridinium derivative of formula A' according to claim 1 being 2,3-(methylenedioxy)-5-ethyl-7-hydroxybenzo[c]phenanthridinium hydrogen sulfate salt.

**7.** The process for preparing the benzo[c]phenanthridinium derivative of formula A' according to claim 1 being 5-methyl-7-hydroxy-8-methoxy-benzo[c]phenanthridinium hydrogen sulfate salt.

**8.** A process for preparing a pharmaceutical composition for the use as an antitumor or anti-platelet agent, characterized by admixture or presentation of the compound of formula A' prepared according to one or more of claims 1 - 7 with pharmaceutically acceptable carriers or additives.

## Patentansprüche

### Patentansprüche für folgende Vertragsstaaten : DE, FR, GB, IT, NL, SE

1. Benzo[c]phenanthridinium-Derivate der allgemeinen Formel A':

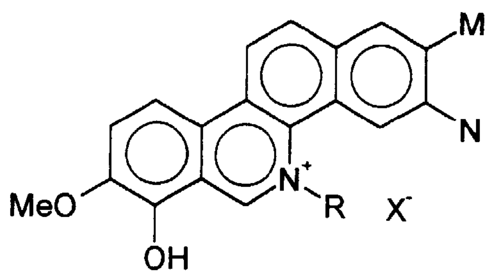

(A')

worin M und N jeweils eine Hydroxyl- oder eine $C_1$-$C_5$-Alkoxygruppe darstellen, oder M und N gleichzeitig Wasserstoffatome darstellen oder zusammen eine Methylendioxygruppe bilden, $X^-$ $HSO_4^-$ oder $H_2PO_4^-$ ist und R eine $C_1$-$C_5$-Alkylgruppe darstellt.

2. Benzo[c]phenanthridinium-Derivate nach Anspruch 1, worin die $C_1$-$C_5$-Alkoxygruppe ausgewählt wird aus der Gruppe die besteht aus einer Methoxy-, einer Ethoxy-, einer n-Propoxy- und einer Isopropoxy-Gruppe.

3. Benzo[c]phenanthridinium-Derivate nach Anspruch 1, worin M und N gemeinsam eine Methylendioxygruppe bilden.

4. Benzo[c]phenanthridinium-Derivate nach Anspruch 3, worin die $C_1$-$C_5$-Alkylgruppe aus der Gruppe ausgewählt wird, die besteht aus einer Methyl-, einer Ethyl-, einer n-Propyl- und einer Isopropyl-Gruppe.

5. Benzo[c]phenanthridinium-Derivate der Formel A' nach Anspruch 1, das 2,3-(Methylendioxy)-5-methyl-7-hydroxy-8-methoxybenzo[c]phenanthridinium-Hydrogensulfatsalz ist.

6. Benzo[c]phenanthridinium-Derivate der Formel A' nach Anspruch 1, das 2, 3- (Methylendioxy) -5-ethyl-7-hydroxy-8-methoxybenzo[c]phenanthridinium-Hydrogensulfatsalz ist.

7. Benzo[c]phenanthridinium-Derivate der Formel A' nach Anspruch 1, das 5-Methyl-7-hydroxy-8-methoxybenzo [c] phenanthridinium-Hydrogensulfatsalz ist.

8. Pharmazeutische Zusammensetzung zur Verwendung als Antitumormittel oder als Antithrombozytenmittel, das als wirksamen Bestandteil eine Verbindung nach irgendeinem der vorhergehenden Patentansprüche und pharmazeutisch verträgliche Träger und/oder Additive umfaßt.

### Patentansprüche für folgenden Vertragsstaat : ES

1. Verfahren zur Herstellung eines Benzo[c]phenanthridinium-Derivates der allgemeinen Formel A':

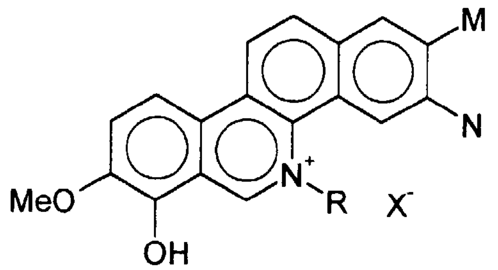

(A')

worin M und N jeweils eine Hydroxyl- oder eine $C_1$-$C_5$-Alkoxygruppe darstellen, oder M und N gleichzeitig Wasserstoffatome darstellen oder zusammen eine Methylendioxygruppe bilden, $X^-HSO_4$ oder $H_2PO_4^-$ ist, und R eine $C_1$-$C_5$-Alkylgruppe darstellt, worin eine Verbindung der allgemeinen Formel C:

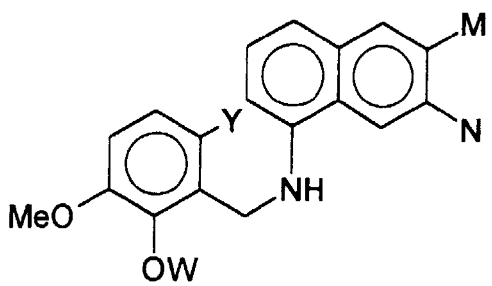

(C)

worin M und N jeweils eine Hydroxyl- oder eine $C_1$-$C_5$-Alkoxygruppe darstellen oder M und N gleichzeitig Wasserstoffatome bilden oder zusammen eine Methylendioxygruppe bilden; Y ein Halogenatom ist und W eine Schutzgruppe ist, einer Ringschlußreaktion in Gegenwart einer organischen Zinnverbindung und dann einer oxidativen Aromatisierungsreaktion unterworfen wird, um eine Verbindung der allgemeinen Formel D zu erhalten;

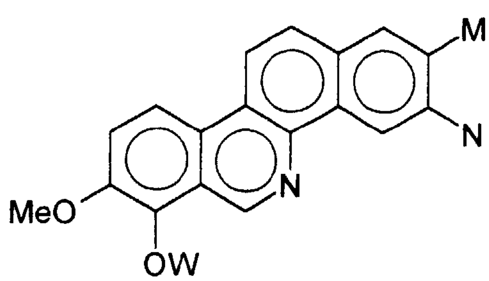

(D)

worin M und N jeweils eine Hydroxyl- oder eine $C_1$-$C_5$-Alkoxygruppe darstellen, oder M und N gleichzeitig Wasserstoffatome bilden oder zusammen eine Methylendioxygruppe bilden; und W eine Schutzgruppe ist, worin die so gebildete Verbindung D mit einem N-Alkylierungsmittel umgesetzt wird, um eine N-Alkylierung der erwähnten Verbindung durchzuführen; und worin die so gebildete N-alkylierte Verbindung einer Entfernungsreaktion der Schutzgruppe unterworfen wird und mit $H_2SO_4$ oder $H_3PO_4$ behandelt wird, um das gewünschte Salzprodukt zu erhalten.

2. Verfahren zur Herstellung der Benzo[c]phenanthridinium-Derivate nach Anspruch 1, worin die $C_1$-$C_5$-Alkoxygruppe ausgewählt wird aus der Gruppe die besteht aus einer Methoxy-, einer Ethoxy-, einer n-Propoxy- und einer Isopropoxy-Gruppe.

3. Verfahren zur Herstellung der Benzo[c]phenanthridinium-Derivate nach Anspruch 1, worin M und N gemeinsam

eine Methylendioxygruppe bilden.

4. Verfahren zur Herstellung der Benzo[c]phenanthridinium-Derivate nach Anspruch 3, worin die $C_1$-$C_5$-Alkylgruppe aus der Gruppe ausgewählt wird, die besteht aus einer Methyl-, einer Ethyl-, einer n-Propyl- und einer Isopropyl-Gruppe.

5. Verfahren zur Herstellung des Benzo[c]phenanthridinium-Derivates der Formel A' nach Anspruch 1, das 2,3-(Methylendioxy)-5-methyl-7-hydroxy-8-methoxybenzo[c]phenanthridinium-Hydrogensulfatsalz ist.

6. Verfahren zur Herstellung des Benzo[c]phenanthridinium-Derivates der Formel A' nach Anspruch 1, das 2,3-(Methylendioxy)-5-ethyl-7-hydroxy-8-methoxybenzo[c]phenanthridinium-Hydrogensulfatsalz ist.

7. Verfahren zur Herstellung des Benzo[c]phenanthridinium-Derivates der Formel A' nach Anspruch 1, das 5-Methyl-7-hydroxy-8-methoxybenzo[c]-phenanthridinium-Hydrogensulfatsalz ist.

8. Verfahren zur Herstellung einer pharmazeutischen Zusammensetzung zur Verwendung als Antitumormittel oder als Antithrombozytenmittel, dadurch gekennzeichnet, daß die Verbindung der Formel A' nach einem oder mehreren der Patentansprüche 1 bis 7 mit pharmazeutisch verträglichen Trägern und/oder Additiven vermischt oder formuliert wird.

## Revendications

### Revendications pour les Etats contractants suivants : DE, FR, GB, IT, NL, SE

1. Dérivé de benzo[c]phénanthridinium de formule générale A':

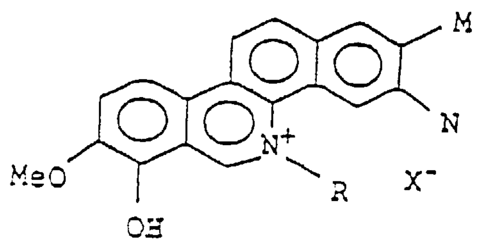

(A')

dans laquelle M et N représentent individuellement un groupe hydroxyle ou alcoxy en $C_1$-$C_5$, ou M et N représentent simultanément un atome d'hydrogène ou forment ensemble un groupe méthylènedioxy, $X^-$ représente $HSO_4^-$ ou $H_2PO_4^-$; et R représente un groupe alkyle en $C_1$-$C_5$.

2. Dérivé de benzo[c]phénanthridinium selon la revendication 1, dans lequel le groupe alcoxy en $C_1$ - $C_5$ est choisi dans le groupe constitué par les groupes méthoxy, éthoxy, n-propoxy et isopropoxy.

3. Dérivé de benzo[c]phénanthridinium selon la revendication 1, dans lequel M et N forment ensemble un groupe méthylènedioxy.

4. Dérivé de benzo[c]phénanthridinium selon la revendication 3, dans lequel le groupe alkyle en $C_1$-$C_5$ est choisi dans le groupe constitué par les groupes méthyle, éthyle, n-propyle et isopropyle.

5. Dérivé de benzo[c]phénanthridinium de formule A' selon la revendication 1, qui est le sel hydrogénosulfate de 2,3-(méthylènedioxy)-5-méthyl-7-hydroxy-8-méthoxybenzo[c]phénanthridinium.

6. Dérivé de benzo[c]phénanthridinium de formule A' selon la revendication 1, qui est le sel hydrogénosulfate de 2,3-(méthylènedioxy)-5-éthyl-7-hydroxy-8-méthoxybenzo[c]phénanthridinium.

7. Dérivé de benzo[c]phénanthridinium de formule A' selon la revendication 1, qui est le sel hydrogénosulfate de

5-méthyl-7-hydroxy-8-méthoxybenzo[c]phénanthridinium.

8. Composition pharmaceutique à utiliser comme agent antitumoral et antiplaquettaire comprenant, comme principe actif, un composé selon l'une quelconque des revendications précédentes et des véhicules et/ou additifs pharmaceutiquement acceptables.

**Revendications pour l'Etat contractant suivant : ES**

1. Procédé pour préparer un dérivé de benzo[c]phénanthridinium de formule générale A':

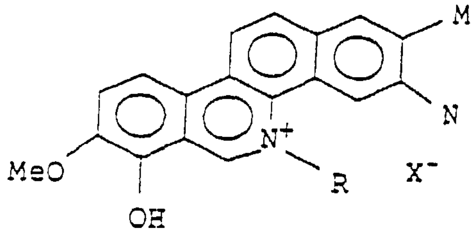

(A')

dans laquelle M et N représentent individuellement un groupe hydroxyle ou alcoxy en $C_1$-$C_5$, ou M et N représentent simultanément un atome d'hydrogène ou forment ensemble un groupe méthylènedioxy, $X^-$ représente $HSO_4^-$ ou $H_2PO_4^-$; et R représente un groupe alkyle en $C_1$-$C_5$;
dans laquelle on soumet un composé de formule générale C:

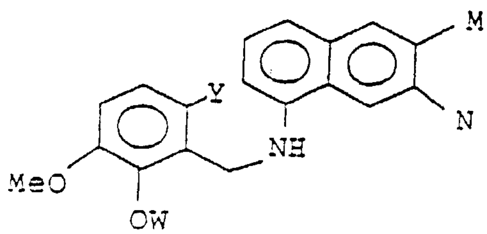

(C)

dans laquelle M et N représentent individuellement un groupe hydroxyle ou alcoxy en $C_1$-$C_5$, ou M et N représentent simultanément un atome d'hydrogène ou forment ensemble un groupe méthylènedioxy, Y représente un atome d'halogène et W représente un groupe protecteur,
à une réaction de fermeture de cycle, en présence d'un composé organique à base d'étain, puis à une réaction d'aromatisation oxydante, pour obtenir un composé de formule générale D:

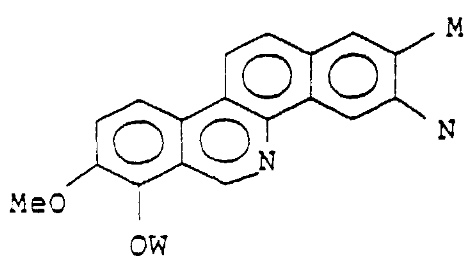

(D)

dans laquelle M et N représentent individuellement un groupe hydroxyle ou alcoxy en $C_1$-$C_5$, ou M et N représentent simultanément un atome d'hydrogène ou forment ensemble un groupe méthylènedioxy, et W représente un groupe protecteur, et dans lequel on fait réagir le composé D ainsi formé avec un agent de N-alkylation pour effectuer une N-alkylation dudit composé; et dans lequel le composé N-alkylé ainsi formé est soumis à une opération d'élimination du groupe protecteur et traité avec $H_2SO_4$ ou $H_3PO_4$ pour former le produit de type sel attendu.

**2.** Procédé pour préparer le dérivé de benzo[c]phénanthridinium selon la revendication 1 dans lequel le groupe alcoxy en $C_1$-$C_5$ est choisi dans le groupe constitué par les groupes méthoxy, éthoxy, n-propoxy et isopropoxy.

**3.** Procédé pour préparer le dérivé de benzo[c]phénanthridinium selon la revendication 1 dans lequel M et N forment ensemble un groupe méthylènedioxy.

**4.** Procédé pour préparer le dérivé de benzo[c]phénanthridinium selon la revendication 3 dans lequel le groupe alkyle en $C_1$-$C_5$ est choisi dans le groupe constitué par les groupes méthyle, éthyle, n-propyle et isopropyle.

**5.** Procédé pour préparer le dérivé de benzo[c]phénanthridinium de formule A' selon la revendication 1 qui est le sel hydrogénosulfate de 2,3-(méthylènedioxy)-5-méthyl-7-hydroxy-8-méthoxybenzo[c]phénanthridinium.

**6.** Procédé pour préparer le dérivé de benzo[c]phénanthridinium de formule A' selon la revendication 1 qui est le sel hydrogénosulfate de 2,3-(méthylènedioxy)-5-éthyl-7-hydroxy-8-méthoxybenzo[c]phénanthridinium.

**7.** Procédé pour préparer le dérivé de benzo[c]phénanthridinium de formule A' selon la revendication 1 qui est le sel hydrogénosulfate de 5-méthyl-7-hydroxy-8-méthoxybenzo[c]phénanthridinium.

**8.** Procédé pour préparer une composition pharmaceutique à utiliser comme agent antitumoral et antiplaquettaire caractérisé par le mélange ou la présentation du composé de formule A' préparé selon l'une quelconque des revendications 1-7 avec des véhicules et/ou additifs pharmaceutiquement acceptables.